Europäisches Patentamt

European Patent Office

Office européen des brevets

(11) Veröffentlichungsnummer: **0 492 209 A1**

# EUROPÄISCHE PATENTANMELDUNG

(21) Anmeldenummer: **91120930.2**

(51) Int. Cl.5: **B01D 61/44**, C07C 303/02

(22) Anmeldetag: **06.12.91**

(30) Priorität: **22.12.90 DE 4041571**

(43) Veröffentlichungstag der Anmeldung:
**01.07.92 Patentblatt 92/27**

(84) Benannte Vertragsstaaten:
**BE CH DE FR GB IT LI NL**

(71) Anmelder: **BASF Aktiengesellschaft
Carl-Bosch-Strasse 38
W-6700 Ludwigshafen(DE)**

(72) Erfinder: **Voss, Hartwig, Dr.
Weinbietring 19
W-6710 Frankenthal(DE)**
Erfinder: **Schneider, Rolf, Dr.
Feldbergstrasse 21
W-6800 Mannheim 1(DE)**

(54) **Verfahren zur Freisetzung von organischen Sulfonsäuren.**

(57) Verfahren zur Freisetzung organischer Sulfonsäuren aus wäßrigen Lösungen ihrer Salze, in dem man die wäßrige Organosulfonat-Lösung mit Hilfe einer Elektrodialysezelle, die aus Diluatkammern KD, Säurekammern KS und Basekammern KB entsprechend der Sequenz I

$$(MB/KS/MA/KD/MK/KB)_n \qquad I$$

aufgebaut ist,
in der
MB     für eine bipolare Ionenaustauschermembran,
MA     für eine Anionenaustauschermembran,
MK     für eine Kationenaustauschermembran und
n     für eine Zahl von 1 bis 500 stehen,
elektrodialysiert.

EP 0 492 209 A1

Bei fast allen industriellen Verfahren zur Herstellung organischer Sulfonsäuren fallen diese in Form ihrer wäßrigen Salzlösungen, also als Sulfonate, beispielsweise als Alkalimetall- oder Ammoniumsulfonate an (vgl. hierzu z.B. US-A 2 818 426, DE-A 960 196, US-A 2 727 057, US-A 2 693 489, US-A 2 810 747). Zur Isolierung der freien Sulfonsäuren sind bislang sehr aufwendige chemische verfahren erforderlich, beispielsweise das Einleiten wasserfreien Chlorwasserstoffgases in alkoholische Alkalisulfonatlösungen zwecks Ausfällung des betreffenden Alkalimetallchlorids, welches dann von der freigesetzten Sulfonsäure abfiltriert wird (s. z.B. US-A 4 499 028 und US-A 4 696 773). Als Folge dieser aufwendigen Isolierungsverfahren sind die freien organischen Sulfonsäuren in der Regel erheblich teurer als ihre Salze.

Der vorliegenden Erfindung lag daher die Aufgabe zugrunde, ein im industriellen Maßstab praktikables verfahren zu finden, das es ermöglicht, organische Sulfonsäuren auf wirtschaftliche Weise aus ihren wäßrigen Salzlösungen freizusetzen und dabei, die bei den herkömmlichen Isolierungsverfahren anfallenden, großen Mengen an Abfallsalzen entweder zu reduzieren oder besser ganz zu vermeiden. Das Verfahren zur Freisetzung der Sulfonsäuren sollte, wenn möglich, auch noch einen zusätzlichen Reinigungseffekt bei der jeweils freizusetzenden Sulfonsäure bewirken, d.h. im Zuge von deren Freisetzung sollte auch eine Abreicherung der gegebenenfalls im eingesetzten Sulfonat-Salz enthaltenen Verunreinigungen stattfinden.

Dementsprechend wurde ein Verfahren zur Freisetzung organischer Sulfonsäuren aus wäßrigen Lösungen ihrer Salze gefunden, das dadurch gekennzeichnet ist, daß man die wäßrige Organosulfonat-Lösung mit Hilfe einer Elektrodialysezelle, die aus Diluatkammern KD, Säurekammern KS und Basekammern KB, entsprechend der Sequenz I

(MB/KS/MA/KD/MK/KB)$_n$      I

aufgebaut ist,
in der
  MB      für eine bipolare Ionenaustauschermembran,
  MA      für eine Anionenaustauschermembran,
  MK      für eine Kationenaustauschermembran und
  n       für eine Zahl von 1 bis 500 stehen,
elektrodialysiert.

Im erfindungsgemäßen Verfahren wird die organische Sulfonsäure aus der wäßrigen Lösung ihrer Salze, vorzugsweise aus der wäßrigen Lösung eines einzelnen ihrer Salze, mit Hilfe eines Elektrodialyse-Dreikreis-Prozesses freigesetzt. Dabei wird so vorgegangen, daß man die wäßrige Sulfonsäuresalzlösung durch die Diluatkammern KD, Wasser oder eine verdünnte Lösung der freizusetzenden organischen Sulfonsäure durch die Säurekammern KS und Wasser oder eine verdünnte Lösung der dem im Salz enthaltenen Kation entsprechenden Base durch die Basekammern KB einer Elektrodialysezelle mit der Sequenz I

(MB/KS/MA/KD/MK/KB)$_n$      I

leitet, worin MB, MA, MK, KS, KB und KD die obengenannte Bedeutung haben und n eine ganze Zahl von 1 bis 500, vorzugsweise von 1 bis 300 und besonders bevorzugt von 50 bis 300 ist und die Elektrodialyse mit Stromdichten von 1 bis 500 mA/cm$^2$, vorzugsweise von 1 bis 300 mA/cm$^2$ und besonders bevorzugt von 1 bis 100 mA/cm$^2$ durchführt, wobei das hierfür benötigte elektrische Feld mittels zweier Elektroden an den Enden des Membranstapels angelegt wird. Der Membranstapel ist zweckmäßigerweise von den beiden Elektrodenräumen, Kathoden- und Anodenraum, durch je eine zusätzliche Ionenaustauschermembran, vorzugsweise eine Kationen- oder Anionenaustauschermembran, abgetrennt. Die beiden Elektrodenräume werden während des Betriebs der Elektrodialysezelle permanent mit einer Elektrodenspüllösung, beispielsweise Lösungen von Schwefelsäure oder vorzugsweise Lösungen der freizusetzenden Base oder der betreffenden Sulfonsäure, umspült. Bei der Auswahl des Materials der die Elektrodenräume vom Membranstapel abtrennenden Ionenaustauschermembranen ist selbstverständlich darauf zu achten, daß dieses Material gegenüber der jeweils verwendeten Elektrodenspüllösung chemisch stabil ist.

Bei der Elektrodialyse der organischen Sulfonsäuresalz-Lösung in der oben beschriebenen Elektrodialysezelle laufen, schematisch dargestellt, die folgenden Vorgänge ab:

Aufgrund des an den Membranstapel angelegten, elektrischen Feldes migrieren aus der in die Diluatkammern KD eingespeisten Organosulfonsäuresalzlösung das Organosulfonatanion und das betreffende Gegenkation in Richtung von Anode bzw. Kathode, wobei das Anion nach Passieren der Anionenaustauschermembran MA in die Säurekammer KS und das Kation nach Passieren der Kationenaustauschermembran MK in die Basekammer KB gelangen. Bei diesem Vorgang bleiben im Diluat gelöste, elektrisch neutrale Verunreinigungen der Organosulfonsäuresalzlösung in der Diluatkammer zurück. Währenddessen wird in der bipolaren Membran MB unter Einwirkung des angelegten, elektrischen Feldes Wasser, rein formal dargestellt, in Protonen und Hydroxylanionen zerlegt, worauf die Protonen auf ihrem Weg zur Kathode in die Säurekammern KS und die Hydroxylanionen auf ihrem Weg zur Anode in die Basekammern KB wandern, wo sie sich mit den dorthin migrierten Organosulfonatanionen bzw. mit den betreffenden Gegenkationen zur freien Organosulfonsäure bzw. zur freien Base kombinieren. Aus den auf diese Weise in den Säure- und Basekammern gebildeten Organosulfonsäure- bzw. Baselösungen, können die Organosulfonsäuren und selbstverständlich auch die betreffenden Basen, je nach Wunsch in Substanz oder in Form von Lösungen gewünschter Konzentration nach herkömmlichen Methoden, beispielsweise durch Verdampfung des Wassers und gegebenenfalls eine sich anschließende Kristallisation, gewonnen werden.

Mit Hilfe des erfindungsgemäßen Verfahrens können praktisch alle Organosulfonsäuren aus ihren Salzen freigesetzt werden, vorausgesetzt, die Organosulfonatanionen und die betreffenden Gegenkationen sind unter den angewandten Elektrodialysebedingungen in der Lage, die eingesetzten Anionen- bzw. Kationenaustauschermembranen zu durchdringen. Diese Fähigkeit ist zum einen abhängig von der Permeabilität der betreffenden Membranen, zum anderen von der Struktur und insbesondere von der Molekülgröße der betreffenden Organosulfonatanionen und der Gegenkationen. Üblicherweise können Organosulfonsäuren mit Molekulargewichten bis ca. 200 Dalton mit Hilfe des erfindungsgemäßen Verfahrens aus ihren Salzen freigesetzt werden. Gleiches gilt in etwa auch für die betreffenden Gegenkationen.

Die nach dem erfindungsgemäßen Verfahren freisetzbaren Organosulfonsäuren können aliphatischer, cycloaliphatischer, aromatischer und araliphatischer Natur sein und unter den Elektrodialysebedingungen inerte Substituenten, wie Halogenatome, Hydroxylgruppen oder Ethergruppen, tragen. Selbstverständlich ist das erfindungsgemäße Verfahren auch zur Freisetzung ungesättigter, aliphatischer Sulfonsäuren geeignet. Beispielsweise können die folgenden Sulfonsäuren nach dem erfindungsgemäßen Verfahren aus ihren Salzen freigesetzt werden:
Benzolsulfonsäure, p-Toluolsulfonsäure, Benzylsulfonsäure, 3-Oxopropan-1-sulfonsäure, 4-Hydroxybutan-2-sulfonsäure, 3-Hydroxybutan-1-sulfonsäure, 2-Hydroxyethan-1-sulfonsäure, 2-Hydroxypropan-1-sulfonsäure, 2-Hydroxy-but-3-en-1-sulfonsäure, Vinylsulfonsäure, 3-Hydroxypropan-1,2-disulfonsäure, Methansulfonsäure, Ethansulfonsäure, Trifluormethansulfonsäure, Methandisulfonsäure, 2-Chlorethan-1-sulfonsäure, Allylsulfonsäure, Prop-2-in-sulfonsäure.

Beispiele für freisetzbare Basen sind:
Alkalihydroxyde, quaternäre Ammoniumhydroxyde, tertiäre, sekundäre und primäre Amine sowie Ammoniak.

Als Ionenaustauschermembranen können an sich bekannte, handelsübliche Ionenaustauschermembranen verwendet werden, die z.B. eine Dicke von 0,1 bis 1 mm und einen Porendurchmesser von 1 bis 30 µm oder eine gelartige Struktur haben. Die Anionenaustauschermembranen sind üblicherweise aus einem Matrixpolymer aufgebaut, beispielsweise einem Poly-Styrol-Divinyl-benzol-Harz, das chemisch gebundene, kationische Gruppen enthält, beispielsweise Ammonium-, Alkyl- oder Dialkylammonium-Gruppen, wohingegen bei den Kationenaustauschermembranen das Matrixpolymer, chemisch gebundene, anionische Gruppen trägt, beispielsweise Carboxylat- oder Sulfonat-Gruppen. Ionenaustauschermembranen der genannten Art sind z.B. unter den Bezeichnungen SELEMION® (Asahi Glass), NEOSEPTA® (Tokoyama Soda) oder IONAC® (Ionac Chemical Company), im Handel erhältlich. Die bipolaren Membranen sind asymmetrisch aufgebaut und tragen auf der einen Seite anionische Gruppen und auf der anderen Seite der Membran kationische Gruppen. Die bipolaren Membranen können beispielsweise durch Zusammenlegen, Verkleben bzw. Verbinden von Kationen- und Anionenaustauschermembranen, beispielsweise gemäß den Verfahren von EP-A 193 959 und WO 89/01059, oder als "single-film"-Membran, wie z.B. in US-A 4 057 481 beschrieben, hergestellt werden.

Als Elektrodialysezellen werden im erfindungsgemäßen Verfahren an sich bekannte, mit Austauschermembranen und Dichtrahmen ausgerüstete Vorrichtungen verwendet, die über bis zu 1500, vorzugsweise bis zu 900, parallel zueinander angeordnete Kammern, entsprechend 500 bzw. 300 Sequenzen, verfügen. Die Membranabstände betragen üblicherweise 0,4 bis 2 mm.

Das erfindungsgemäße Verfahren kann sowohl diskontinuierlich als auch kontinuierlich ausgeübt werden und wird in der Regel bei Temperaturen von 0 bis 100°C, vorzugsweise 10 bis 60°C und bei einem Druck von 1 bis 10 bar, bevorzugt bei Atmosphärendruck betrieben. Die Strömungsgeschwindigkeit der durch die Diluat-, Säure- und Basekammern geleiteten Lösungen wird im allgemeinen auf eine Geschwindigkeit von 0,001 bis 2,0, vorzugsweise von 0,01 bis 0,1 m/s eingestellt.

Die im erfindungsgemäßen verfahren eingesetzten Organosulfonsäuresalzlösungen haben üblicherweise eine Konzentration von 0,1 bis 4, bevorzugt von 0,5 bis 2 mol Sulfonat/l. Mit Hilfe des vorliegenden Verfahrens können Organosulfonsäurelösungen mit einem Sulfonsäuregehalt von bis zu 3 mol Sulfonsäure/kg Lösung gewonnen werden. Analog dazu können in den Basekammern des erfindungsgemäßen Verfahrens Baselösungen mit einer Basekonzentration von bis zu 3 mol Base/kg Lösung erhalten werden. Bei der Erzeugung derart konzentrierter Baselösungen ist es möglich, daß sich ein im Falle der elektrodialytischen Spaltung eines Organosulfonsäureammoniumsalzes freigesetztes Amin infolge Überschreitens der Löslichkeitsgrenze als organische Phase in der Basekammer abscheidet.

Die nach dem erfindungsgemäßen Verfahren erhältlichen Sulfonsäurelösungen, aber auch die Baselösungen, fallen in der Regel in sehr reiner Form an, da gegebenenfalls im eingesetzten Sulfonsäuresalz enthaltene, elektrisch neutrale Verunreinigungen im Zuge der Freisetzung der Sulfonsäuren und Basen entweder vollständig abgetrennt oder doch zumindest stark abgereichert werden. Somit ist das erfindungsgemäße Verfahren nicht nur zur Freisetzung und zusätzlichen Reinigung der Sulfonsäuren vorteilhaft, es liefert nebenher auch noch sehr reine Baselösungen, die vielfältigen Verwendungszwecken zugeführt werden können.

Organische Sulfonsäuren haben ein breites Spektrum von Anwendungen, beispielsweise in ihrer Funktion als Säure zur pH-Erniedrigung und als saure Katalysatoren, aber auch als Zwischenprodukte für Arzneimittel- und Farbstoffsynthesen. Beispielhaft sei hierzu auf Ullmanns Encyklopädie der technischen Chemie, 4. Aufl., Band 7, S. 199 bis 202, Verlag Chemie, Weinheim 1979 und die Ullmann's Encyclopedia of Industrial Chemistry, Vol. A3, S. 507 bis 537, VCH Verlagsgesellschaft, Weinheim 1985, verwiesen.

Beispiele

Allgemeine Elektrodialysebedingungen:

Es wurde eine Laborelektrodialysezelle mit folgendem Aufbau eingesetzt:
KA/(Sequenz I)$_{n=2}$/MB/KK

KA: Anodenkammer mit integrierter Anode

KK: Kathodenkammer mit integrierter Kathode.

Die effektive Fläche einer Membran betrug 3,14 cm$^2$ und der Membranabstand 1 cm. Die Kammern KS, KD und KB hatten separate Pumpkreisläufe, die Kammern KK und KA waren über einen gemeinsamen Pumpkreislauf miteinander verbunden. Es wurde bei einer Temperatur von 35 bis 38°C gearbeitet. Die Temperatureinstellung erfolgte mittels in die Pumpkreisläufe integrierter Wärmeaustauscher. Die Elektrodialysen wurden diskontinuierlich und mit einem konstanten Strom von 300 mA durchgeführt.

Bei den Elektrodialysen wurden z.T. mit elektrisch neutralen, organischen Stoffen verunreinigte Organosulfonsäuresalzlösungen, z.T. reine Organosulfonsäuresalzlösungen durch die Diluatkammern KD geleitet. In die Säurekammern KS und die Basekammern KB wurde Wasser oder eine verdünnte Lösung der jeweils freizusetzenden Sulfonsäure bzw. Base eingespeist. Durch die Elektrodenräume KA und KK wurde entweder verdünnte Natronlauge oder verdünnte Natriumsulfatlösung gepumpt.

Es wurden die Kationen- bzw. Anionenaustauschermembranen SELEMION® CMV bzw. AMV (Asahi Glass) verwendet. Die bipolaren Membranen waren aus monopolaren Membranen nach dem Verfahren von EP-A 193 959 hergestellt worden.

Beispiel 1

Eine wäßrige Natrium-2-hydroxyethansulfonat-Lösung, die durch die Umsetzung äquimolarer Mengen Ethylenoxid mit einer 40 gew.-%igen Natriumhydrogensulfitlösung bei 40°C im Autoklaven hergestellt worden war und als Verunreinigung Ethylenglykol enthielt, wurde gemäß den Allgemeinen Elektrodialysebedingungen elektrodialysiert. Vor ihrer Einspeisung in die Elektrodialysevorrichtung war die 49 gew.-%ige Sulfonsäuresalzlösung mit Wasser im Verhältnis 1:1 (v/v) verdünnt worden. Die Elektrodialysezeit betrug 14 h, als Elektrodenspüllösung wurde 5 gew.-%ige Natriumsulfatlösung verwendet. Die Ergebnisse dieser Elektrodialyse sind in Tabelle 1 aufgelistet.

Tabelle 1

| | Einsatz | | | Austrag | | |
|---|---|---|---|---|---|---|
| | Basekreis | Säurekreis | Diluatkreis | Basekreis | Säurekreis | Diluatkreis |
| Menge (g) | 130 | 144 | 100 | 155 | 183 | 34 |
| $RSO_3Na$ (mol/kg) | - | - | 1,80 | - | - | 0,059 |
| $RSO_3H$ (mol/kg) | - | - | - | - | 0,94 | - |
| NaOH (mol/kg) | 0,03 | - | - | 1,16 | - | - |
| Ethylenglykol (Gew.-%) | - | - | 0,2 | - | - | 0,5 |

Beispiel 2

Eine reine Natriummethansulfonatlösung wurde gemäß den Allgemeinen Elektrodialysebedingungen während eines Zeitraums von 25,33 h elektrodialysiert. Die Einsatz- und Austragskonzentrationen dieser Elektrodialyse sind in Tabelle 2 aufgeführt. Als Elektrodenspüllösung wurde eine 1,2 gew.-%ige Natriumhydroxydlösung verwendet.

Tabelle 2

| | Einsatz | | | Austrag | | |
|---|---|---|---|---|---|---|
| | Basekreis | Säurekreis | Diluatkreis | Basekreis | Säurekreis | Diluatkreis |
| Menge (g) | 106,6 | 183,3 | 179,5 | 155,2 | 245,4 | 64,0 |
| $CH_3SO_3Na$ (mol/kg) | - | - | 1,91 | 0,009 | 0,01 | 0,001 |
| $CH_3SO_3H$ (mol/kg) | - | 0,014 | - | - | 1,39 | - |
| NaOH (mol/kg) | 0,12 | - | - | 2,21 | - | - |

Beispiel 3

Bei der gemäß den Allgemeinen Elektrodialysebedingungen durchgeführten Elektrodialyse einer Abfall-Natriummethansulfonatlösung wurden die in Tabelle 3 dargestellten Ergebnisse erzielt. Die eingesetzte Natriummethansulfonatlösung war durch die Umsetzung einer Ammoniummethansulfonatlösung mit Natronlauge und Strippen des dabei freigesetzten Ammoniaks erhalten worden. Die Ammoniummethansulfonatlösung war ihrerseits bei der Synthese eines Polyetherdiamins aus dem entsprechenden Polyether-bis-methansulfonat durch dessen Umsetzung mit Ammoniak entstanden und enthielt als Verunreinigung das betreffende Polyetherdiamin.

Die Elektrodialysezeit betrug in diesem Versuch 24 h, als Elektrodenspüllösung wurde 1,1 gew.-%ige Natriumhydroxydlösung verwendet.

Tabelle 3

| | Einsatz | | | Austrag | | |
|---|---|---|---|---|---|---|
| | Basekreis | Säurekreis | Diluatkreis | Basekreis | Säurekreis | Diluatkreis |
| Menge (g) | 103,3 | 286,3 | 192,5 | 156,4 | 339,5 | 81,7 |
| $CH_3SO_3Na$ (mol/kg) | - | - | 1,64 | - | - | 0,05 |
| $CH_3SO_3H$ (mol/kg) | - | 0,06 | - | - | 0,94 | - |
| NaOH (mol/kg) | 0,11 | - | 0,24 | 2,3 | - | - |
| Polyetherdiamin (Gew.-%) | - | - | 3,3 | - | - | 7,6 |

Beispiel 4

EP 0 492 209 A1

Eine wäßrige Lösung einer Mischung der Natriumsalze der 2-Hydroxy-but-3-en-1-sulfonsäure und 1-Hydroxy-but-3-en-2-sulfonsäure wurde gemäß den Allgemeinen Elektrodialysebedingungen elektrodialysiert. Diese Sulfonat-Lösung war bei der Umsetzung von Vinyloxiran mit Natriumhydrogensulfitlösung analog dem in Beispiel 1 beschriebenen Verfahren entstanden. Die Einsatz- und Austragskonzentrationen sind der Tabelle 4 zu entnehmen. Die Elektrodialysezeit in diesem Versuch betrug 19 h, als Elektrodenspüllösung wurde eine 1 gew.-%ige Natriumhydroxydlösung eingesetzt.

Tabelle 4

|  | Einsatz | | | Austrag | | |
|---|---|---|---|---|---|---|
|  | Basekreis | Säurekreis | Diluatkreis | Basekreis | Säurekreis | Diluatkreis |
| Menge (g) | 205,2 | 221,4 | 150,0 | 235,4 | 292,3 | 49,3 |
| $RSO_3Na$ (mol/kg) | - | - | 1,72 | - | - | 0,08 |
| $RSO_3H$ (mol/kg) | - | - | - | - | 0,85 | - |
| NaOH (mol/kg) | 0,15 | - | - | 1,19 | - | - |

**Patentansprüche**

1. Verfahren zur Freisetzung organischer Sulfonsäuren aus wäßrigen Lösungen ihrer Salze, dadurch gekennzeichnet, daß man die wäßrige Organosulfonat-Lösung mit Hilfe einer Elektrodialysezelle, die aus Diluatkammern KD, Säurekammern KS und Basekammern KB entsprechend der Sequenz I

   $(MB/KS/MA/KD/MK/KB)_n$        I

   aufgebaut ist,
   in der
       MB      für eine bipolare Ionenaustauschermembran,
       MA      für eine Anionenaustauschermembran,
       MK      für eine Kationenaustauschermembran und
       n       für eine Zahl von 1 bis 500 stehen,
   elektrodialysiert.

6

Europäisches
Patentamt

EUROPÄISCHER RECHERCHENBERICHT

Nummer der Anmeldung

EP    91 12 0930
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | CHEMICAL ABSTRACTS, vol. 71, no. 12, 22. September 1969, Columbus, Ohio, US; abstract no. 56451F, T.D. PRISHLETSOVA ET AL.: 'SPECTROPHOTOMETRIC DETERMINATION OF IRON AND AMMONIUM TRACES IN BENZENESULFONIC ACID' Seite 533 ; Spalte 2 ; * Zusammenfassung * --- | 1 | B01D61/44 C07C303/02 |
| Y | CHEMIE-INGENIEUR-TECNNIK Bd. 61, Nr. 5, Mai 1989, WEINHEIM,DE Seiten 428 - 429; S. SRIDHAR: 'ELEKTRODIALYS MIT BIPOLAREN MEMBRANEN' * das ganze Dokument * --- | 1 | |
| A | FR-A-2 646 421 (C.N.R.S.) * Zusammenfassung; Ansprüche 1,4,5; Abbildungen 4,5; Beispiel 3 * --- | 1 | |
| A | US-A-4 781 809 (FALCONE) * Zusammenfassung; Abbildung 2 * * Spalte 6, Zeile 11 - Zeile 68 * --- | 1 | **RECHERCHIERTE SACHGEBIETE (Int. Cl.5)** |
| A | US-A-2 829 095 (ODA) * Spalte 1, Zeile 50 - Spalte 2, Zeile 33 * * Spalte 3, Zeile 74 - Spalte 4, Zeile 9 * * Beispiel 2 * --- | 1 | B01D C07C |
| A | CHEMICAL ABSTRACTS, vol. 109, no. 16, 17. Oktober 1988, Columbus, Ohio, US; abstract no. 131270E, SOTODANI ET AL.: 'PREPARATION OF ACID-TYPE AMINOSULFONIC ACID WITH USE OF ELECTRODIALYSIS' Seite 117 ; Spalte 1 ; * Zusammenfassung * & PATENT ABSTRACTS OF JAPAN vol. 12, no. 299 (C-520)15. August 1988 & JP-A-63 069 990 ( KAO CORP ) 30. März 1988 * Zusammenfassung * | 1 | |

Der vorliegende Recherchenbericht wurde für alle Patentansprüche erstellt

| Recherchenort | Abschlußdatum der Recherche | Prüfer |
|---|---|---|
| DEN HAAG | 14 FEBRUAR 1992 | HOORNAERT P.G.R.J. |

Europäisches
Patentamt

**EUROPÄISCHER RECHERCHENBERICHT**

Nummer der Anmeldung

EP    91 12 0930
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| | --- | | |
| A | PATENT ABSTRACTS OF JAPAN vol. 13, no. 119 (C-579)23. März 1989 & JP-A-63 293 189 ( TOKUYAMA SODA CO LTD ) 30. November 1988 * Zusammenfassung * & WORLD PATENTS INDEX LATEST Section Ch, Week 8903, Derwent Publications Ltd., London, GB; Class A, AN 89-018564 * Zusammenfassung * | 1 | |
| | --- | | |
| A | PATENT ABSTRACTS OF JAPAN vol. 14, no. 96 (C-069)22. Februar 1990 & JP-A-1 304 190 ( KAO CORP ) 7. Dezember 1989 * Zusammenfassung * | 1 | |
| | ----- | | |
| | | | **RECHERCHIERTE SACHGEBIETE (Int. Cl.5)** |

Der vorliegende Recherchenbericht wurde für alle Patentansprüche erstellt

| Recherchenort | Abschlußdatum der Recherche | Prüfer |
|---|---|---|
| DEN HAAG | 14 FEBRUAR 1992 | HOORNAERT P.G.R.J. |